# FASCICULE DE BREVET EUROPEEN

(11) **EP 2 954 902 B1**
(45) Date de publication et mention de la délivrance du brevet: **30.12.2020**
(21) Numéro de dépôt: 15172031.5
(22) Date de dépôt: 15.06.2015
(51) Int. Cl.: A61K 36/752, A61K 36/9066, A61Q 11/00, A61K 8/97, A61K 36/28, A61K 36/73, A61P 1/02

(54) **COMPOSITION À BASE D'EXTRAIT DE PÉPIN DE PAMPLEMOUSSE, D'EXTRAIT DE FEUILLE D'ALCHÉMILLE, D'EXTRAIT DE STEVIA ET DE CURCUMINE**
ZUSAMMENSETZUNG AUF DER BASIS VON GRAPEFRUITKERN-, FRAUENMANTEL-, STEVIA- UND KURKUMINEXTRAKT
COMPOSITION COMPRISING GRAPEFRUIT SEED EXTRACT, ALCHEMILLA LEAF EXTRACT, STEVIA EXTRACT AND CURCUMIN

(30) Priorité: 13.06.2014 FR 1455409
(43) Date de publication de la demande: 16.12.2015
(73) Titulaire: Haddad, Laurent, 92200 Neully-Sur-Seine (FR); Stemmer, Vivian, 75116 Paris (FR)
(72) Inventeur: Haddad, Laurent, 92200 Neully-Sur-Seine (FR); Stemmer, Vivian, 75116 Paris (FR)
(74) Mandataire: Casalonga

(56) Documents cités:
- WO-A1-00/74668
- WO-A1-2005/067865
- WO-A1-2007/077210
- WO-A1-2008/074978
- WO-A1-2011/084744
- WO-A1-2012/116445
- WO-A1-2013/049507
- WO-A2-02/074274
- WO-A2-2006/109344
- WO-A2-2008/001325
- WO-A2-2010/070675
- US-B1- 6 395 309
- DATABASE WPI Week 201240 Thomson Scientific, London, GB; AN 2011-P50514 XP002735436, & KR 2011 0122908 A (LEE C Y) 14 novembre 2011 (2011-11-14)
- DATABASE WPI Week 200951 Thomson Scientific, London, GB; AN 2009-L99007 XP002735437, & JP 2009 167204 A (KOKURITSU YOBO EISEI KENKYUSHO) 30 juillet 2009 (2009-07-30)
- DATABASE WPI Week 201439 Thomson Scientific, London, GB; AN 2014-L53696 XP002735438, & CN 103 735 473 A (HE X) 23 avril 2014 (2014-04-23)

## Description

La présente invention concerne une composition comprenant au moins un extrait de pépin de pamplemousse, au moins un extrait de feuille d'alchémille, au moins un extrait de stevia et au moins de la curcumine.

L'invention porte également sur ladite composition se trouvant sous la forme d'un dispositif médical ou d'une composition cosmétique ainsi que sur son utilisation dans le traitement des affections de la cavité buccale choisies parmi les infections fongiques.

La présente invention porte aussi sur le fait que ladite composition est utilisée pour son effet antalgique et permet donc de diminuer et/ou de soulager les douleurs liées aux affections de la cavité buccale décrites précédemment.

L'invention concerne également l'utilisation de ladite composition pour la préservation de la flore buccale résidente.

La cavité buccale est généralement le siège de multiples affections, notamment de saignements, d'inflammations telles que la gingivite et la parodontite qui peuvent être notamment causées par la bactérie *Prevotella melaninogenica,* de lésions affectant la muqueuse buccale et la langue, telles que les aphtes buccaux et autres ulcérations buccales, ainsi que d'infections bactériennes, fongiques (ou mycoses buccales) telles que la candidose, et d'infections virales.

En effet, les aphtes buccaux et autres ulcérations buccales sont des lésions affectant la muqueuse buccale et/ou la langue qui se traduisent le plus souvent par des enflures, des plaies ou des modifications structurelles plus ou moins importantes de la bouche, de la langue et des lèvres et peuvent s'avérer douloureux, désagréables et gênants pour la mastication, la déglutition ou la phonation.

Typiquement, les aphtes apparaissent préférentiellement au niveau des gencives, de la langue ainsi que sur le bord interne des lèvres et des joues et se présentent souvent sous la forme d'enflures arrondies ou ovales recouvertes par une membrane blanchâtre ou jaunâtre pouvant être entourée d'un liséré rougeâtre. Les aphtes peuvent résulter d'une mauvaise hygiène bucco-dentaire, d'une allergie, d'un mauvais entretien de prothèses dentaires, d'une influence hormonale particulière (périodes menstruelles ou de grossesses), d'une carence en vitamines, de l'ingestion de certains aliments tels que les noix ou le gruyère, de certains médicaments, du stress ou encore de maladies provoquant l'apparition d'aphtes telles que la maladie de Behcet qui sont souvent d'origine virale.

D'autres ulcérations affectant fréquemment la muqueuse buccale et/ou la langue peuvent être par exemple des lésions ayant un aspect blanchâtre. De telles lésions sont souvent liées à une anomalie de la kératinisation et peuvent aussi bien revêtir un caractère bénin qu'un caractère précancéreux.

Parmi les infections fongiques, la candidose est une mycose buccale causée par la présence d'une levure dénommée *Candida albicans.* La candidose se manifeste le plus souvent par la présence de plaques, pouvant être de couleur crème, jaune blanchâtre ou rouge, située au niveau de la langue et de la muqueuse buccale. La candidose est courante chez les personnes portant notamment un appareil dentaire, les nouveau-nés ainsi que les personnes ayant un système immunitaire amoindri.

Par ailleurs, la cavité buccale peut être aussi le siège d'affections essentiellement liées à la mauvaise haleine (halitose), la sensibilité gingivale et/ou dentaire ou être le lieu de prolifération de nombreuses bactéries telles que le *Streptococcus mutans* qui est le principal responsable de l'apparition de caries.

En particulier, la sensibilité gingivale et/ou dentaire peut notamment survenir en cas de rétractation des gencives autour des dents ou de perte de tissu gingival. Par exemple, la perte du tissu gingival peut survenir à la suite d'un brossage de dents trop vigoureux ou d'une absence de brossage et d'utilisation régulière d'un fil dentaire. Cette perte conduit à exposer la racine de la dent et les canaux reliant la racine et à son centre nerveux. Ainsi cette perte va entraîner une douleur à la suite d'un stimulus thermique, c'est-à-dire lorsque les dents et/ou la gencive se retrouvent en contact avec un liquide froid ou chaud.

De la même façon, le *Streptococcus mutans* est une bactérie qui adhère fortement aux tissus dentaires et aux autres bactéries présentes dans la cavité buccale. Cette bactérie a une tendance à produire un biofilm polysaccharide sur les dents ou une plaque dentaire pouvant conduire à l'apparition de caries.

Ces différents types de désordre de la cavité buccale induisent naturellement des sensations inconfortables et/ou des douleurs chez l'être humain. Le document WO2006/109344 décrit des compositions à base de plantes, dont un extrait de pépin de pamplemousse, pour le traitement de maladies de la cavité buccale.

A cet effet, de nombreuses compositions pharmaceutiques et/ou cosmétiques, rincées ou non, destinées à l'hygiène bucco-dentaire ont été développées dans l'état de la technique pour traiter ces différents types de désordres afin de réduire ou d'éliminer les sensations d'inconfort et/ou les douleurs afférentes. Cependant, les compositions d'hygiène bucco-dentaire disponibles à l'heure actuelle présentent encore un certain nombre d'inconvénients.

En effet, de telles compositions présentent souvent des valeurs de pH trop acides par rapport à la valeur du pH dans la cavité buccale qui est généralement neutre du fait de la présence abondante de la salive.

De plus, ces compositions provoquent fréquemment une douleur plus ou moins vive chez l'utilisateur lors de leur contact avec la muqueuse buccale et/ou la langue, notamment lorsqu'elles sont appliquées au niveau des saignements, des lésions ou des plaques causées par les infections fongiques.

Enfin, ces compositions sont la plupart du temps difficiles à appliquer, notamment au niveau des plaies et/ou des lésions, en raison de l'état physiologique aqueux de la cavité buccale. En effet, la salive a tendance à empêcher la fixation de la composition sur la surface à traiter ce qui a pour effet de diminuer leur efficacité. En d'autres termes, de telles compositions n'adhèrent pas suffisamment à la muqueuse buccale et/ou la langue et ont tendance à être éliminées trop rapidement avant de pouvoir être pleinement efficaces. Cette absence d'adhérence est notamment observée dans les compositions d'hygiène bucco-dentaire non rincées.

Ainsi il existe un réel besoin de mettre à disposition des compositions qui sont notamment capables de traiter efficacement le ou les désordres de la cavité buccale mentionnés précédemment tout en présentant un pH proche de celui de la salive et en minimisant la douleur ressentie au moment de leur application.

Il existe également un réel besoin de mettre à disposition des compositions qui sont en outre capables d'adhérer facilement aux différents endroits à traiter de la cavité buccale.

Au vu de ce qui précède, la présente invention a notamment pour objet une composition comprenant i) au moins un extrait de pépin de pamplemousse, ii) au moins un extrait de feuille d'alchémille, iii) au moins un extrait de stevia et iv) au moins de la curcumine pour son utilisation dans le traitement des affections de la cavité buccale choisies parmi les infections fongiques causées par la présence du Candida albicans.

Ainsi la composition selon l'invention comporte une association synergique de substances pléiotropes de façon à obtenir les propriétés décrites.

En effet, la composition selon l'invention permet de traiter efficacement les désordres de la cavité buccale tels que décrits précédemment tout en respectant le pH dans la cavité buccale. En particulier, le pH de la composition varie de 6,8 à 7,8, de préférence de 7,3 à 7,7.

En particulier, la composition n'est pas irritante d'un point de vue cutanée ce qui permet d'allier innocuité et efficacité.

Plus particulièrement, l'application de la composition selon l'invention sur les surfaces à traiter de la cavité buccale provoque des

Par ailleurs, la composition selon l'invention présente également l'avantage de pouvoir former, au moment de son application, un film capable d'adhérer sur la surface des différents endroits à traiter dans la cavité buccale, notamment au niveau de la muqueuse buccale et de la langue.

L'adhésion du film ainsi formé est favorisée par l'état physiologique aqueux de la cavité buccale grâce à un système épaississant.

L'adhérence du film permet de prolonger le temps d'action de la composition sur la surface à traiter. Autrement dit, la composition selon l'invention n'est pas éliminée trop rapidement dans la cavité buccale.

La composition selon l'invention permet donc d'obtenir un effet bioadhésif amélioré au niveau de la surface à traiter au sein de la cavité buccale.

Au sens de la présente invention, le caractère « bioadhésif » désigne la propriété d'une composition à adhérer sur un tissu biologique, notamment une muqueuse dans la cavité buccale, en particulier à l'épithélium de la muqueuse, afin de créer une nouvelle interface sur la muqueuse et de s'y maintenir pendant un temps plus ou moins long. Le mécanisme de l'effet bioadhésif est décrit dans la littérature scientifique.

L'effet bioadhésif permet de pouvoir utiliser efficacement la composition en mode non rincé.

La composition selon l'invention présente l'avantage d'être conforme au Règlement (CE) 1223/2009.

Cependant, étant donné que la composition va être en contact avec la muqueuse buccale, il a été choisi de ne sélectionner que des matières premières cosmétiques :
- ayant un grade alimentaire ou pharmaceutique,
- étant composées de substances autorisées en tant qu'additifs alimentaires par le Règlement 1129/2011 ou ayant un recul d'utilisation dans l'industrie alimentaire.
- étant composées de substances autorisées en tant qu'additifs alimentaires par le Règlement 1129/2011 ou ayant un recul d'utilisation dans l'industrie alimentaire.

De plus, la composition selon l'invention présente l'avantage de pouvoir s'étaler sur l'ensemble de la ou les surfaces à traiter de la cavité buccale ce qui contribue à améliorer son efficacité. Par exemple, la composition peut s'étaler facilement sur l'ensemble de la lésion à traiter ce qui favorise sa cicatrisation.

Ainsi la composition de la présente invention présente un bon pouvoir mouillant et hydratant.

La composition selon l'invention présente donc l'avantage de traiter efficacement les affections de la cavité buccale et de diminuer et/ou de soulager les douleurs liées à de telles affections.

En particulier lorsque la cavité buccale est le siège d'affections, la composition selon l'invention peut être directement appliquée sur la ou les surface(s) à traiter, en particulier sur la ou les lésion(s) affectant la muqueuse buccale et/ou la langue, afin de permettre leur cicatrisation et diminuer les sensations désagréables de douleurs et/ou de gênes induites par de telles affections.

Ainsi la composition selon l'invention présente aussi une action antalgique réalisée par protection de l'atteinte tissulaire et saturation des nocirécepteurs.

La composition selon l'invention présente aussi l'avantage de traiter efficacement les infections fongiques telles que la candidose.

En particulier, lorsque la cavité buccale est le siège de la candidose, la composition selon l'invention peut être directement appliquée sur la ou les surface(s) à traiter afin de diminuer et/ou d'éliminer la levure dénommée *Candida albicans* responsable de cette infection ainsi que de leur prolifération.

En d'autres termes, la composition selon l'invention présente l'avantage de présenter des propriétés antimycosiques tout en maintenant la flore buccale résidente.

De plus, grâce à son caractère hydrosoluble, la composition peut être insérée dans différentes compositions d'hygiène bucco-dentaire, notamment dans des dentifrices et des bains de bouche.

Il en résulte que la composition selon l'invention présente l'avantage de pouvoir être utilisée dans le domaine thérapeutique, notamment pour traiter les affections de la cavité buccale ou des affections différentes, ainsi que dans le domaine cosmétique, notamment pour traiter cosmétiquement la cavité buccale.

La présente invention concerne également la composition telle que décrite précédemment sous forme de dispositif médical.

L'invention est aussi relative à la composition telle que décrite précédemment sous forme d'une composition cosmétique.

De même, l'invention a également pour objet la composition telle que décrite précédemment pour son utilisation dans le domaine thérapeutique.

En particulier, l'invention a trait à la composition pour son utilisation dans le traitement des affections de la cavité buccale choisies parmi les infections fongiques causées par la présence du Candida albicans.

Un autre objet de la présente invention porte sur la composition telle que décrite précédemment pour son utilisation dans la diminution ou le soulagement de la douleur causée par les affections de la cavité buccale choisies parmi les infections fongiques causées par la présence du Candida albicans.

L'invention est aussi relative à l'utilisation de la composition telle que décrite précédemment pour préserver la flore résidente de la cavité buccale.

Conformément à la présente invention, dans ce qui va suivre, et à moins d'une indication contraire, les bornes d'un domaine de valeurs sont comprises dans ce domaine.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Comme indiqué ci-avant, la composition selon l'invention comprend i) au moins un extrait de pépin de pamplemousse.

L'extrait de pamplemousse utilisé dans la composition selon l'invention comprend essentiellement des flavonoïdes et de l'acide ascorbique éventuellement de la glycérine, des extraits d'écorce ou pépins de pamplemousse et un extrait d'agave bleu.

De préférence, l'extrait de pépin de pamplemousse utilisé dans la composition selon l'invention présente un grade alimentaire ou pharmaceutique.

De préférence, la composition selon l'invention comprend de l'extrait de pépin de pamplemousse dans une teneur allant de 1 à 40% en poids, plus préférentiellement dans une teneur allant de 10 à 35% en poids par rapport au poids total de la composition

Comme indiqué ci-avant, la composition selon l'invention comprend également ii) au moins un extrait de feuille d'alchémille.

L'extrait de feuille d'alchémille utilisé dans la composition selon l'invention est préférentiellement obtenu à partir d'un procédé d'extraction dans lequel on soumet des feuilles d'alchémille sèches,

De préférence, l'extrait est fabriqué à partir de 170 g de feuilles séchées pour 1 kg d'extrait final.

L'extraction peut se dérouler éventuellement sous agitation à une température allant de 10 à 30°C pendant une durée pouvant aller de 8 à 20 heures.

Préférentiellement, l'extrait de feuille d'alchémille utilisé dans la composition est un extrait de feuille d'alchémille commune (ou *Alchemilla vulgaris L).*

De préférence, l'extrait de feuille d'alchémille est une composition pouvant contenir de 1,5% à 100% en poids de feuille d'alchémille.

En particulier, l'extrait de feuille d'alchémille utilisable dans la composition selon l'invention peut être une solution hydroalcoolique à base d'extrait de feuille d'alchémille commune.

De préférence, la composition selon l'invention comprend de l'extrait de feuille d'alchémille dans une teneur allant de 1 à 10% en poids, plus préférentiellement dans une teneur allant de 4 à 6,5% en poids par rapport au poids total de la composition.

Comme indiqué ci-avant, la composition selon l'invention comprend également iii) au moins un extrait de stevia.

L'extrait de stevia utilisé dans la composition selon l'invention est préférentiellement obtenu à partir d'un procédé d'extraction à l'eau déminéralisée dans lequel on soumet les parties aériennes (feuilles) de stevia fraîches ou sèches, broyées ou pulvérisées.

L'extraction peut se dérouler éventuellement sous agitation à une température allant de 95°C à 99°C pendant une durée pouvant aller jusqu'à 4 heures.

Les solutions obtenues suite à l'extraction peuvent être éventuellement concentrées et/ou séchées en étuve sous vide et refroidies à température ambiante.

Le procédé d'extraction peut comprendre une étape de cristallisation après l'étape de séchage à l'aide d'un solvant à base d'éthanol et d'eau.

Préférentiellement, l'extrait de stevia est un extrait de *Stevia rebaudiana,* notamment un extrait de feuille de *Stevia rebaudiana.*

L'extrait de stevia préférentiellement utilisé dans la composition selon l'invention est un extrait de *Stevia rebaudiana* vendu sous la dénomination commerciale Extrait de Stevia 97% REB-A par la société Quimdis.

De préférence, la composition selon l'invention comprend l'extrait de stevia dans une teneur allant de 0,025 à 0,1% en poids, plus préférentiellement dans une teneur allant de 0,045 à 0,065% en poids par rapport au poids total de la composition.

Comme indiqué ci-avant, la composition selon l'invention comprend également iv) au moins de la curcumine.

La curcumine [1,7-bis-(4-hydroxy-3-méthoxyphényl)-1,6-heptadiène-3,5-dione] est un dérivé de polyphénol hydrophobe dérivé de l'épice curcuma.

De préférence, la composition selon l'invention comprend de la curcumine dans une teneur allant de 0,0001 à 0,01% en poids, plus particulièrement de 0,0001 % à 0,001% en poids, par rapport au poids total de la composition.

Selon un mode de réalisation de l'invention, la composition comprend au moins un extrait de pépin de pamplemousse, au moins un extrait de feuille d'alchémille commune (ou *Alchemilla vulgaris L*)*,* au moins un extrait de feuille de *Stevia rebaudiana* et au moins de la curcumine, notamment de la curcumine à 90%.

De préférence, la composition selon l'invention comprend au moins un agent gélifiant.

Ainsi la composition selon l'invention se trouve avantageusement sous la forme d'un gel, en particulier d'un gel non rincé, ce qui facilite sa préhension ainsi que son application et son adhérence sur la ou les surface(s) à traiter au sein de la cavité buccale.

L'agent gélifiant permet notamment d'améliorer l'adhérence de la composition sur la surface à traiter dans la cavité buccale et de retarder sa dissolution. Le temps d'action de la composition est par conséquent prolongé ce qui améliore son efficacité.

Grâce à son comportement pseudoplastique, l'agent gélifiant permet aussi de:
- diminuer la tension superficielle de la composition selon l'invention par rapport à la surface à traiter dans la cavité buccale lorsqu'elle est soumise à une contrainte physique ce qui conduit à une amélioration de son étalement.
- d'augmenter la tension de superficielle de la composition selon l'invention par rapport à la surface à traiter dans la cavité buccale lorsque celle-ci n'est soumise à aucune contrainte physique ce qui permet d'améliorer sa bioadhésivité.

De préférence, la composition selon l'invention comprend au moins un agent gélifiant non ionique.

Par agent gélifiant, on entend au sens de la présente invention, un agent capable d'épaissir la composition selon l'invention.

La viscosité de la composition est de préférence mesurée à l'aide d'un rhéomètre Brookfield MV-III à température ambiante à une vitesse de cisaillement de 10 rpm.

De préférence, l'agent gélifiant non ionique est choisi parmi les polysaccharides, en particulier les gommes de xanthane, les gommes de guar, les alginates et les polymères de celluloses comme l'hydroxyéthylcellulose.

Plus préférentiellement, la composition selon l'invention comprend au moins une gomme de xanthane.

De préférence, l'agent gélifiant est présent dans la composition selon l'invention dans une teneur allant de 1 à 2% en poids et plus particulièrement dans une teneur allant de 1 à 1,3% en poids par rapport au poids total de la composition.

Avantageusement, la composition selon l'invention peut comprendre en outre de l'hyaluronate de sodium.

Le hyaluronate de sodium permet de favoriser la formation d'un film sur la surface à traiter dans la cavité buccale.

De préférence, le hyaluronate de sodium peut être présent dans la composition selon l'invention dans une teneur allant de 0,1 à 1,5% en poids et plus particulièrement dans une teneur allant de 0,85 à 1,1% en poids par rapport au poids total de la composition.

Selon un mode de réalisation préféré, la composition est sous forme d'un gel et comprend au moins un extrait de pépin de pamplemousse, au moins un extrait de feuille d'alchémille commune (ou *Alchemilla vulgaris L*)*,* au moins un extrait de feuille de *Stevia rebaudiana,* au moins de la curcumine et au moins un agent gélifiant non ionique.

Conformément à ce mode de réalisation, l'agent gélifiant non ionique est notamment choisi parmi les polysaccharides.

Plus particulièrement, l'agent gélifiant non ionique est une gomme de xanthane.

Conformément à ce mode de réalisation, la composition comprend en outre de l'hyaluronate de sodium.

Selon un mode de réalisation, la composition peut également comprendre au moins un extrait végétal différent des extraits végétaux précédemment décrits.

En particulier, l'extrait végétal est préférentiellement un extrait de clou de girofle, notamment une huile essentielle de clou de girofle.

De préférence, l'extrait végétal peut être présent dans la composition selon l'invention dans une teneur allant de 0,05 à 1% en poids et plus préférentiellement dans une teneur allant de 0,25 à 0,5% en poids par rapport au poids total de la composition.

La composition selon l'invention peut également comprendre au moins une huile, notamment une huile d'origine végétale.

Par huile, on entend tout composé liquide, non aqueux et insoluble dans l'eau à température ambiante (25°C) et à pression atmosphérique (760 mm Hg).

En particulier, l'huile d'origine végétale utilisable dans la composition peut être l'huile d'avocat.

De préférence, l'huile d'origine végétale peut être présente dans la composition selon l'invention dans une teneur allant de 0,1 à 1% en poids et plus préférentiellement dans une teneur allant de 0,35 à 0,60% en poids par rapport au poids total de la composition.

La composition selon l'invention peut en outre comprendre un ou plusieurs additifs choisis parmi des tensioactifs non ioniques, des polymères anioniques, non ioniques, ou leurs mélanges, des huiles essentielles, des agents anti-inflammatoires, des agents de blanchiments, des agents anti-plaque dentaire tel que le chlorure de cétylpyridinium, des agents dispersants ; des agents filmogènes, des agents antioxydants, des édulcorants, des agents antifongiques et antibactériens, des agents apaisants, des agents hydratants et des agents humectants, des agents de lutte contre l'halitose, des agents astringents, anti-oedemateux ; des agents favorisant la croissance cellulaire ; des agents effet « barrière » aux frottements et au pH acide.

La composition selon l'invention présente notamment un pH allant de 6,8 à 7,8, de préférence de 7,3 à 7,7.

De préférence, la composition selon l'invention peut comprendre en outre un ou plusieurs tensioactifs cationiques, en particulier le chlorure de cétylpyridinium.

Selon un mode de réalisation particulier, la composition comprend :
- au moins un extrait de pépin de pamplemousse,
- au moins un extrait de feuille d'alchémille (ou *Alchemilla vulgaris L),*
- au moins un extrait de *Stevia rebaudiana,*
- au moins de la curcumine,
- au moins un agent gélifiant non ionique, en particulier la gomme de xanthane,
- de l'acide hyaluronique.

La présente invention a également pour objet la composition telle que décrite précédemment sous la forme d'un dispositif médical.

En particulier, la composition selon l'invention se trouve sous la forme d'un dispositif médical tel que défini selon la Directive 2007/47/CE.

La présente invention a également pour objet la composition telle que décrite précédemment sous la forme d'un dispositif médical.

En particulier, la composition selon l'invention se trouve sous la forme d'un dispositif médical tel que défini selon la Directive 2007/47/CE.

Selon un mode de réalisation, le dispositif médical comprend la composition selon l'invention.

L'invention est aussi relative à la composition telle que décrite ci-avant pour son utilisation dans le domaine thérapeutique.

Ainsi l'invention concerne aussi un procédé de traitement des affections de la cavité buccale choisies parmi les infections fongiques causées par la présence du Candida albicans consistant à appliquer la composition telle que décrite ci-avant sur la ou les surface(s) à traiter de la cavité buccale.

Au sens de la présente invention, la cavité buccale est délimitée par le palais, le plancher buccal, les joues, les lèvres ainsi que la luette et les voûtes palatines de part et d'autre de la luette.

La composition selon l'invention est utilisée pour le traitement des affections de la cavité buccale choisies parmi les infections fongiques.

La composition selon l'invention présente notamment l'avantage de pouvoir former un film capable de correctement adhérer à la lésion de la muqueuse buccale et/ou de la langue ce qui améliore sa cicatrisation. Ainsi la composition forme un film protecteur entre la lésion à traiter et la salive ce qui favorise un effet antalgique.

La composition selon l'invention est utilisée pour le traitement d'infections fongiques causées par la présence d'une levure dénommée *Candida albicans.*

Autrement dit, la composition selon l'invention peut être utilisée pour le traitement de la candidose.

En particulier, la composition selon l'invention est utilisée pour diminuer la prolifération de *Candida albicans.*

La composition selon l'invention peut être utilisée dans le traitement d'une ou plusieurs affections telles que décrites précédemment, notamment les lésions de la cavité buccale, les infections microbiennes et l'halitose.

La composition peut donc être utilisée dans le traitement de plusieurs affections à la fois.

Plus préférentiellement, la composition selon l'invention est utilisée pour le traitement des affections de la cavité buccale choisies parmi les saignements, les aphtes buccaux, la sensibilité gingivale, l'halitose, les infections microbiennes, notamment les infections bactériennes, fongiques ou les mycoses et les infections virales.

Ainsi la composition selon l'invention permet de diminuer voire d'éliminer les douleurs liées aux affections de la cavité buccale grâce à son effet antalgique.

En d'autres termes, l'invention est aussi relative à une composition utilisée pour le traitement ou le soulagement des douleurs liées aux affections de la cavité buccale telles que décrites précédemment.

En effet, la composition peut générer un procédé antalgique par protection de l'atteinte tissulaire et saturation des nocirécepteurs.

Autrement dit, l'invention est aussi relative à une composition selon l'invention utilisée pour son action antalgique liée aux affections de la cavité buccale.

Par ailleurs, la composition selon l'invention peut être utilisée dans un domaine thérapeutique différent de celui du secteur dentaire.

En particulier, l'invention vise à proposer une composition telle que décrite précédemment pour son utilisation dans le traitement des affections autre que celles de la cavité buccale.

Plus particulièrement, la composition telle que décrite précédemment peut être utilisée dans le traitement des affections différentes de celles décrites précédemment, en particulier des plaies corporelles non chroniques, c'est-à-dire des plaies destinées à être cicatrisées dans un délai de 4 à 6 semaines après leur apparition.

Selon un mode de réalisation particulier, l'invention concerne l'utilisation *in vitro* de la composition telle que décrite ci-avant contre la prolifération de microorganismes choisis parmi le *Pseudomonas aeruginosa,* le *Staphylococcus aureus,* l'*Escherichia coli,* le *Candida albicans* et *l'Aspergillus brasiliensis.*

La présente invention concerne également la composition telle que décrite précédemment sous la forme d'une composition cosmétique.

En effet, la composition selon l'invention présente l'avantage de pouvoir traiter cosmétiquement la cavité buccale.

En particulier, la composition permet de protéger la muqueuse buccale.

De plus, l'invention a pour objet l'utilisation de la composition selon l'invention pour la préservation de la flore résidente de la cavité buccale.

En d'autres termes, l'invention consiste à utiliser la composition telle que décrite précédemment afin de maintenir l'équilibre de la flore buccale résidente.

De préférence, la composition selon l'invention est utilisée pour assainir la cavité buccale car cette dernière présente une activité antimicrobienne avantageuse tout en respectant la flore buccale résidente.

Selon un mode de réalisation, la composition est appliquée sur la surface à traiter de la cavité buccale.

La composition peut être notamment appliquée pendant une période de trois semaines tous les deux mois, à raison de deux fois par jour.

De préférence, la composition selon l'invention est appliquée sur les lésions de la muqueuse buccale et/ou de la langue, notamment sur les aphtes.

La composition selon l'invention peut être conditionnée dans un flacon pompe.

La composition selon l'invention présente notamment l'avantage de pouvoir être introduite dans de nombreuses compositions d'hygiène buccodentaires telles que des dentifrices, des compositions destinées au polissage et à la brillance de l'émail et des bains de bouche.

De préférence, la composition selon l'invention est notamment incorporée dans des dentifrices et des bains de bouche.

Les exemples suivants servent à illustrer l'invention sans toutefois présenter un caractère limitatif.

### EXEMPLES

### Exemple I :

On prépare les compositions (A) et (B) dont les quantités sont exprimées en pourcentage en poids sauf indications contraires.

| | Composition A | Composition B |
|---|---|---|
| Extrait d'alchémille commune | 5 | 5 |
| Extrait de pépin de pamplemousse (Citrus paradisi peel extract) | 40 | 40 |
| Extrait de *Stevia rebaudiana* | 0,05 | 0,05 |
| Curcumine | 0,001 | 0,001 |
| Extrait de clou de girofle (Eugenia caryophyllus bud oil) | - | 1 |
| Huile d'avocat (Persea gratissima oil) | - | 0,3 |
| Bicarbonate de sodium | 5 | - |
| Alcool | 4 | - |
| Eau | Qsp 100 | Qsp 100 |

Le pH de ces préparations est compris entre 6.8 et 7.8.

On constate que ces préparations peuvent être appliquées sans provoquer une douleur chez l'utilisateur.

### Exemple II :

### I. Préparation de la composition (C) selon l'invention

On prépare la composition (C) selon l'invention conformément au mode opératoire décrit ci-après. Les quantités sont exprimées en pourcentage en poids sauf indications contraires

| | Quantités (% en poids) |
|---|---|
| Gomme de xanthane ⁽¹⁾ | 1,2 |
| Hyaluronate de sodium ⁽²⁾ | 1 |
| Huile de ricin hydrogénée PEG-40 ⁽³⁾ | 0,2 |
| Extrait de clou de girofle (Eugenia caryophyllus bud oil) | 0,4 |
| Bicarbonate de sodium ⁽⁴⁾ | 1,1 (99,47% MA) |
| Extrait d'alchémille commune ⁽⁵⁾ | 5 (>1,5% MA) |
| Sauge bio extrait hydroglycérine 80 ⁽⁶⁾ | 5 |
| Curcumine ⁽⁷⁾ | 0,0003 (90% MA) |
| Extrait de feuille Calendula officinalis ⁽⁸⁾ | 5 |
| Panthénol ⁽⁹⁾ | 0,5 |
| Huile d'avocat (Persea gratissima oil) ⁽¹⁰⁾ | 0,5 (30-50% MA) |
| Extrait de *Stevia rebaudiana* ⁽¹¹⁾ | 0,5 (>97% MA) |
| Xylitol ⁽¹²⁾ | 1 (>98,5% MA) |
| Chlorure de cétyl pyridinium | 1-3% |
| Extrait de pépin de pamplemousse (Citrus paradisi peel extract) ⁽¹³⁾ | 33% |
| Eau | Qs 100 |

| | |
|---|---|
| MA : Matières actives ⁽¹⁾ Vendu sous la dénomination commerciale Keltrol CG ⁽²⁾ Vendu sous la dénomination commerciale Nutrihyl ⁽³⁾ Vendu sous la dénomination commerciale Tagat CH 40, ⁽⁴⁾ Vendu sous la dénomination commerciale Bicarbonate sodium ALIM E500 ⁽⁵⁾ Vendu sous la dénomination commerciale Alchemille extrait hydroalcoolique ⁽⁶⁾ Vendu sous la dénomination commerciale Sauge Bio Extrait Hydroglycérine ⁽⁷⁾ Vendu sous la dénomination commerciale Napture Col Yellow LC 112 ⁽⁸⁾ Vendu sous la dénomination commerciale Calendula Extrait Hydroglycérine 80 ⁽⁹⁾ Vendu sous la dénomination commerciale Dexpanthénol 75% Solution ⁽¹⁰⁾ Vendu sous la dénomination commerciale Huile Avocat Hydrosoluble ⁽¹¹⁾ Vendu sous la dénomination commerciale Extrait de Stevia 97% REB-A ⁽¹²⁾ Vendu sous dénomination commerciale Xivia C ⁽¹³⁾ Vendu sous dénomination commerciale EPP Sirop Agave Abio | |

### II.Mode opératoire

On introduit progressivement un mélange de poudre composé de gomme de xanthane et d'hyaluronate de sodium dans de l'eau, sous forte agitation, jusqu'à l'obtention d'un gel homogène.

On introduit ensuite l'huile de ricin hydrogénée PEG-40, l'extrait de clou de girofle et le bicarbonate de sodium et on mélange sous vive agitation pendant 5 à 10 minutes entre chaque ajout afin d'obtenir un mélange homogène.

On incorpore ensuite le reste des ingrédients en terminant par le chlorure de cétylpyridinium et l'extrait de pépin de pamplemousse puis on mélange sous agitation moyenne de manière à limiter la formation de mousse.

On obtient une composition dont le pH final varie de 7,3 à 7,7.

### III. Etude de la protection antimicrobienne de la composition (C) selon l'invention

Dans cet exemple, la protection antimicrobienne d'une composition (C) selon l'invention est évaluée selon la norme ISO 11930.

Cette évaluation repose sur l'inoculation de la composition (C) sur cinq différentes souches de microorganismes (*Pseudomonas aeruginosa, Staphylococcus aureus, Escherichia coli, Candida albicans* et *Aspergillus brasiliensis*) avec des inocula calibrées.

Le nombre de microorganismes survivants est ensuite mesuré à des intervalles de temps prédéfinis pendant 28 jours sur des géloses de dénombrement.

Les géloses utilisées pour le dénombrement sont la trypto-caséine de soja (TSA) pour les bactéries *Pseudomonas aeruginosa, Staphylococcus aureus* et *Escherichia coli,* le Sabouraud au dextrose (SDA) pour le *Candida albicans* et la pomme de terre et dextrose (PDA) pour *l'Aspergillus brasiliensis.*

Pour chaque temps et chaque souche, le taux de réduction des microorganismes est calculé et comparé aux valeurs minimales requises pour les critères d'évaluation A ou B de la norme ISO 11930.

### IV. Résultats

Les taux de réduction des microorganismes sont indiqués en pourcentage dans le tableau suivant :

| Microorganismes | Taux de réduction des microorganismes (Tx) |
|---|---|
| *Pseudomonas aeruginosa* | Tx > 99,9% des bactéries inoculées dès 7 jours |
| *Staphylococcus aureus* | Tx > 99,9% des bactéries inoculées dès 7 jours |
| *Escherichia coli* | Tx >99,9% des bactéries inoculées dès 7 jours |
| *Candida albicans* | Tx >99,9% des levures inoculées dès 7 jours |
| *Aspergillus brasiliensis* | Tx >99% des moisissures inoculées dèsl4 jours |

Les taux de réduction des microorganismes correspondent aux critères du profil A de la norme ISO 11930.

La composition (C) selon l'invention satisfait donc aux critères d'évaluation du profil A sur la protection antimicrobienne de la norme ISO 11930 et est donc efficace sur le *Pseudomonas aeruginosa,* le *Staphylococcus aureus,* le *Escherichia coli,* le *Candida albicans* et *l'Aspergillus brasiliensis.*

### Exemple III :

Dans cet exemple, on étudie l'activité virucide de la composition (C) selon l'invention, telle que décrite dans l'exemple II, conformément à la norme NF EN 14476.

### I. Méthodologie

L'activité virucide de la composition est testée selon la méthodologie de la norme NF EN 14476.

La souche de virus testée est l'herpès virus de type 1 (HSV1), cultivé sur cellules VERO, à une température de 37°C sous 5% de CO₂.

Cette évaluation se déroule pour des temps de contact de 30, 45 et 60 secondes entre la composition selon l'invention et la suspension virale.

Le titrage est calculé selon la méthode de Spearman-Kärber.

### II. Résultats

La composition (C) selon l'invention est efficace au bout de 45 secondes de contact avec l'herpès virus de type I à une température de 20°C selon la méthodologie de la norme NF EN 14476 avec un taux de réduction de plus de 99,99% du virus.

### Exemple IV :

Dans cet exemple, on étudie l'activité bactéricide de la composition (C) selon l'invention, telle que décrite dans l'exemple II, sur le *Streptococcus mutans* conformément à la norme NF EN 13727.

### Résultats

La composition (C) selon l'invention est efficace au bout d'une minute sur le Streptococcus mutans à une température de 20°C conformément à la méthodologie de la norme NF EN 13727 avec un taux de réduction de plus de 99,99% des bactéries.

### Exemple V :

### I. Préparation de la composition (D) selon l'invention

On prépare la composition (D) selon l'invention conformément au mode opératoire décrit ci-après. Les quantités sont exprimées en pourcentage en poids sauf indications contraires

| | Quantités (% en poids) |
|---|---|
| Hyaluronate de sodium ⁽¹⁾ | 1 |
| Huile de ricin hydrogénée PEG-40 ⁽²⁾ | 0,2 |
| Extrait de clou de girofle (Eugenia caryophyllus bud oil) | 0,4 |
| Bicarbonate de sodium ⁽³⁾ | 1,1 (99,47% MA) |
| Extrait d'alchémille commune ⁽⁴⁾ | 5 (>1,5% MA) |
| Sauge bio extrait hydroglycérine 80 ⁽⁵⁾ | 5 |
| Curcumine ⁽⁶⁾ | 0,0003 (90% MA) |
| Extrait de feuille Calendula officinalis ⁽⁷⁾ | 5 |
| Panthénol ⁽⁸⁾ | 0,5 |
| Huile d'avocat (Persea gratissima oil) ⁽⁹⁾ | 0,5 (30-50% MA) |
| Extrait de *Stevia rebaudiana* ⁽¹⁰⁾ | 0,5 (>97% MA) |
| Xylitol ⁽¹¹⁾ | 1 (>98,5% MA) |
| Chlorure de cétyl pyridinium | 1-3% |
| Extrait de pépin de pamplemousse (Citrus paradisi peel extract) ⁽¹²⁾ | 33% |
| Eau | Qs 100 |

| | |
|---|---|
| MA : Matières actives ⁽¹⁾ Vendu sous la dénomination commerciale Nutrihyl ⁽²⁾ Vendu sous la dénomination commerciale Tagat CH 40, ⁽³⁾ Vendu sous la dénomination commerciale Bicarbonate sodium ALIM E500 ⁽⁴⁾ Vendu sous la dénomination commerciale Alchemille extrait hydroalcoolique ⁽⁵⁾ Vendu sous la dénomination commerciale Sauge Bio Extrait Hydroglycérine ⁽⁶⁾ Vendu sous la dénomination commerciale Napture Col Yellow LC 112 ⁽⁷⁾ Vendu sous la dénomination commerciale Calendula Extrait Hydroglycérine 80 ⁽⁸⁾ Vendu sous la dénomination commerciale Dexpanthénol 75% Solution ⁽⁹⁾ Vendu sous la dénomination commerciale Huile Avocat Hydrosoluble ⁽¹⁰⁾ Vendu sous la dénomination commerciale Extrait de Stevia 97% REB-A ⁽¹¹⁾ Vendu sous dénomination commerciale Xivia C ⁽¹²⁾ Vendu sous dénomination commerciale EPP Sirop Agave Abio | |

### II. Mode opératoire

On introduit progressivement d'hyaluronate de sodium dans de l'eau, sous vive agitation, jusqu'à l'obtention d'un gel homogène.

On introduit ensuite l'huile de ricin hydrogénée PEG-40, l'extrait de clou de girofle et le bicarbonate de sodium et on mélange sous vive agitation pendant 5 à 10 minutes entre chaque ajout afin d'obtenir un mélange homogène.

On incorpore ensuite le reste des ingrédients en terminant par le chlorure de cétylpyridinium et l'extrait de pépin de pamplemousse puis on mélange sous agitation moyenne de manière à limiter la formation de mousse.

On obtient une composition dont le pH final varie de 7,3 à 7,7.

### III. Etude de la protection antimicrobienne de la composition (D) selon l'invention

On étudie la protection antimicrobienne de la composition (D) selon l'invention selon la norme EN 14562.

En particulier, on étudie l'activité antimicrobienne sur le *Candida albicans.*

La composition (D) selon l'invention est efficace au bout d'une minute de contact à une température de 20°C selon la méthodologie de la norme NF EN 14562 avec un taux de réduction de plus de 99,99% des levures.

### Exemple VI :

Dans cet exemple, on étudie les effets buccodentaires de la composition (C) selon l'invention telle que décrite dans l'exemple II sur un panel de 70 volontaires masculins et féminins, utilisateurs ou occasionnels de produits d'hygiène buccodentaires, âgés de 18 à 70 ans, ayant les gencives sensibles et porteurs de couronne(s), de bridge(s) ou d'implant(s) dentaire(s).

### I. Mode opératoire

La composition C) selon l'invention est appliquée à domicile par chaque membre du panel, dans les conditions normales d'utilisation, pendant une durée de 7 jours consécutifs.

0,1 ml de la composition est appliquée au niveau des gencives trois fois par jour pendant une période de sept jours. La composition est massée et n'est pas rincée.

Un examen buccodentaire est réalisé par le même dentiste pour l'ensemble du panel à J1 (T0) avant la 1^{ère} application de la composition et à J7 après sept jours d'utilisation.

L'appréciation des qualités cosmétiques et de l'efficacité de la composition selon l'invention sont répertoriées dans un questionnaire rempli par le panel de volontaires après les sept jours consécutifs d'utilisation.

Le panel est constitué de 70 personnes âgées de 18 à 70 ans.

### II. Résultats

Les pourcentages de volontaires satisfaits selon les critères cosmétiques et d'efficacité de la composition sont regroupés dans le tableau ci-dessous :

| Critères d'évaluation | Pourcentage de volontaires satisfaits |
|---|---|
| Composition s'applique facilement | 94% |
| Texture filmogène permet le maintien de la composition dans la cavité buccale | 71% |
| Composition me convient | 96% |
| Composition adaptée à un usage quotidien | 97% |
| Composition ne donne pas de sensations d'inconfort pendant l'utilisation | 97% |
| Composition ne donne pas de sensations d'inconfort après l'utilisation | 99% |
| Composition convient à mon type de gencive | 99% |
| Composition respecte l'équilibre de la cavité buccale | 100% |
| Composition a rendu mes gencives plus fortes | 97% |
| Composition aide au soulagement | 98% |
| de mes gencives sensibles | |
| Composition a diminué les saignements de mes gencives | 97% |
| Composition a diminué la sensibilité de mes dents au contact thermique (froid ou chaud) | 95% |
| Efficacité immédiate de la composition | 94% |
| Efficacité est durable | 97% |

On constate que la composition selon l'invention présente d'excellentes propriétés buccodentaires.

### Exemple VII :

Dans cet exemple, les effets buccodentaires de la composition (C) selon l'invention, telle que décrite dans l'exemple II, ont été étudiés par un laboratoire de recherche et d'expérimentation externe sur un panel composé de 20 personnes saines dans des conditions normales d'utilisation.

### I. Mode opératoire

Cette étude a été réalisée dans les mêmes conditions que l'étude précédente, c'est-à-dire sur un panel de volontaires composé de 20 personnes saines dans des conditions normales d'utilisation.

L'appréciation des qualités cosmétiques et de l'efficacité de la composition selon l'invention sont répertoriées dans un questionnaire rempli par le panel de volontaires après les sept jours consécutifs d'utilisation.

De plus, les signes cliniques et les sensations d'inconfort sont rapportés directement par les participants au cours de l'étude ou sont indiqués dans la fiche de suivi journalier.

### II.Signes cliniques et sensations d'inconfort répertoriés

Les signes cliniques et les sensations d'inconfort sont répertoriés dans les tableaux ci-après :

### a. Signes cliniques

| Signes cliniques répertoriés | Pourcentage de volontaires concernés |
|---|---|
| Léger dessèchement des lèvres et légère dépapillation linguale | 5% |
| Dépapillation des bords latéraux de la langue | 5% |
| Coloration linguale légère | 5% |

### b. Signes d'inconfort

| Sensations d'inconfort répertoriées | Pourcentage de volontaires concernés |
|---|---|
| Altération importante du sens gustatif après chaque application pendant une heure | 5% |
| Altération légère du sens gustatif après chaque application pendant une heure avec hypersalivation importante pendant 5 à 10 minutes après application de la composition pendant 30 minutes | 5% |
| Sécheresse gingivale modérée après chaque application, permanente | 5% |
| Altération important du sens gustatif et modification modérée de l'haleine après chaque application jusqu'au rinçage | 5% |
| Altération modérée du sens gustatif après chaque application pendant 1 à 2 heures | 5% |
| Hypersalivation modérée après chaque application pendant 5 à 10 minutes | 5% |
| Amertume en bouche | - |

On constate que les sensations d'inconfort ainsi que les signes cliniques négatifs répertoriés sont faibles pour la composition selon l'invention.

### III. Critères d'évaluation buccodentaire de la composition

| Critères d'évaluation | Pourcentage de volontaires satisfaits |
|---|---|
| Texture filmogène permet le maintien de la composition dans la cavité buccale | 85% |
| Composition adaptée à un usage quotidien | 65% |
| Composition ne donne pas de sensations d'inconfort pendant l'utilisation | 75% |
| Composition ne donne pas de sensations d'inconfort après l'utilisation | 85% |
| Composition convient à mon type de gencive | 75% |
| Composition respecte l'équilibre | 80% |
| de la cavité buccale | |
| Composition aide au soulagement de mes gencives sensibles | 65% |
| Composition a diminué les saignements de mes gencives | 70% |
| Composition a diminué la sensibilité de mes dents au contact thermique (froid ou chaud) | 60% |
| Composition diminue la sensation d'halitose | 75% |

On constate que la composition selon l'invention présente dans l'ensemble d'excellentes propriétés buccodentaires.

## Revendications

1. Composition comprenant au moins un extrait de pépin de pamplemousse, au moins un extrait de feuille d'alchémille, au moins un extrait de stevia et au moins de la curcumine pour son utilisation dans le traitement des affections de la cavité buccale choisies parmi les infections fongiques causées par la présence du Candida albicans.

2. Composition pour son utilisation selon la revendication 1 dans le traitement de la candidose.

3. Composition pour son utilisation selon la revendication 1 ou 2, **caractérisée en ce que** l'extrait de feuille d'alchémille est un extrait de feuille d'alchémille commune.

4. Composition pour son utilisation selon l'une quelconque des revendications 1 à 3, **caractérisée en ce que** la stevia correspond à la *Stevia rebaudiana.*

5. Composition pour son utilisation selon l'une quelconque des revendications 1 à 4, **caractérisée en ce qu'**elle est sous forme de gel et comprend au moins un agent gélifiant, de préférence non ionique.

6. Composition pour son utilisation selon la revendication 5, **caractérisée en ce que** l'agent gélifiant non ionique est choisi parmi les polysaccharides, et de préférence une gomme de xanthane.

7. Composition pour son utilisation selon l'une quelconque des revendications précédentes, **caractérisée en ce qu'**elle comprend en outre de l'hyaluronate de sodium.

8. Composition pour son utilisation selon l'une quelconque des revendications précédentes, **caractérisée en ce qu'**elle se trouve sous la forme d'un dispositif médical.

9. Composition pour son utilisation selon l'une quelconque des revendications précédentes dans la diminution ou le soulagement de la douleur causée par les affections de la cavité buccale telles que décrites dans la revendication 1.

10. Composition pour son utilisation selon l'une quelconque des revendications 1 à 9, **caractérisée en ce qu'**elle est adaptée pour être appliquée sur la ou les surfaces à traiter de la cavité buccale.

11. Composition pour son utilisation selon l'une quelconque des revendications 1 à 9 dans la préservation de la flore résidente de la cavité buccale.

## Patentansprüche

1. Zusammensetzung, umfassend mindestens einen Grapefruitkernextrakt, mindestens einen Frauenmantelblattextrakt, mindestens einen Steviaextrakt und mindestens Kurkumin für ihre Verwendung bei der Behandlung der Erkrankungen der Mundhöhle, ausgewählt aus den Pilzinfektionen, die durch die Anwesenheit von Candida albicans hervorgerufen werden.

2. Zusammensetzung für ihre Verwendung nach Anspruch 1 bei der Behandlung der Candidose.

3. Zusammensetzung für ihre Verwendung nach Anspruch 1 oder 2, **dadurch gekennzeichnet, dass** der Frauenmantelblattextrakt ein Extrakt aus den Blättern des gemeinen Frauenmantels ist.

4. Zusammensetzung für ihre Verwendung nach einem der Ansprüche 1 bis 3, **dadurch gekennzeichnet, dass** die Stevia der *Stevia rebaudiana* entspricht.

5. Zusammensetzung für ihre Verwendung nach einem der Ansprüche 1 bis 4, **dadurch gekennzeichnet, dass** sie in Form von Gel vorliegt und mindestens ein vorzugsweise nichtionisches Geliermittel umfasst.

6. Zusammensetzung für ihre Verwendung nach Anspruch 5, **dadurch gekennzeichnet, dass** das nichtionische Geliermittel aus den Polysacchariden ausgewählt und vorzugsweise ein Xanthan ist.

7. Zusammensetzung für ihre Verwendung nach einem der vorangehenden Ansprüche, **dadurch gekennzeichnet, dass** sie ferner Natriumhyaluronat umfasst.

8. Zusammensetzung für ihre Verwendung nach einem der vorangehenden Ansprüche, **dadurch gekennzeichnet, dass** sie sich in Form eines Medizinprodukts befindet.

9. Zusammensetzung für ihre Verwendung nach einem der vorangehenden Ansprüche bei der Verringerung oder Linderung von Schmerz, verursacht durch die Erkrankungen der Mundhöhle wie solchen, die in Anspruch 1 beschrieben sind.

10. Zusammensetzung für ihre Verwendung nach einem der Ansprüche 1 bis 9, **dadurch gekennzeichnet, dass** sie für das Auftragen auf die zu behandelnden Fläche(n) der Mundhöhle geeignet ist.

11. Zusammensetzung für ihre Verwendung nach einem der Ansprüche 1 bis 9 zur Bewahrung der Standardflore der Mundhöhle.

## Claims

1. Composition comprising at least one grapefruit seed extract, at least one alchemilla leaf extract, at least one stevia extract and at least curcumin for use in the treatment of oral cavity conditions selected from fungal infections caused by the presence of Candida albicans.

2. Composition for use according to claim 1 in the treatment of candidiasis.

3. Composition for use according to claim 1 or 2, **characterised in that** the alchemilla leaf extract is an *Alchemilla vulgaris* leaf extract.

4. Composition for use according to any one of claims 1 to 3, **characterised in that** the stevia consists of *Stevia rebaudiana.*

5. Composition for use according to any one of claims 1 to 4, **characterised in that** it is in the form of a gel and comprises at least one gelling agent, preferably non-ionic.

6. Composition for use according to claim 5, **characterised in that** the non-ionic gelling agent is selected from polysaccharides, and preferably a xanthan gum.

7. Composition for use according to any one of the preceding claims, **characterised in that** it further comprises sodium hyaluronate.

8. Composition for use according to any one of the preceding claims, **characterised in that** it is in the form of a medical device.

9. Composition for use according to any one of the preceding claims in the reduction and the relief of pain caused by oral cavity conditions as described in claim 1.

10. Composition for use according to any one of claims 1 to 9, **characterised in that** it is adapted to be applied on the surface(s) to be treated of the oral cavity.

11. Composition for use according to any one of claims 1 to 9 in the preservation of the resident flora of the oral cavity.
